# FASCICULE DE BREVET EUROPEEN

(11) **EP 0 364 540 B1**
(45) Date de publication et mention de la délivrance du brevet: **29.12.1993**
(21) Numéro de dépôt: 89903755.0
(22) Date de dépôt: 21.03.1989
(51) Int. Cl.: A61B 5/03

(54) **DISPOSITIF DE MESURE DE LA PRESSION D'UN LIQUIDE, A USAGE MEDICAL**
VORRICHTUNG ZUM MESSEN DES DRUCKS EINER FLÜSSIGKEIT, INSBESONDERE FÜR MEDIZINISCHEN GEBRAUCH
DEVICE FOR MEASURING THE PRESSURE OF A LIQUID, FOR MEDICAL USE

(30) Priorité: 21.03.1988 FR 8803845
(43) Date de publication de la demande: 25.04.1990
(73) Titulaire: FORTUNO, Richard, F-33650 Cabanac-et-Villagrains (FR)
(72) Inventeur: FORTUNO, Richard, F-33650 Cabanac-et-Villagrains (FR)
(86) Numéro de dépôt international: FR8900125
(87) Numéro de publication internationale: WO8909018

(56) Documents cités:
- WO-A-85/02333
- US-A- 3 856 009
- US-A- 4 003 370
- US-A- 4 609 370
- Proceedings of the IEEE, vol.65, no.5, May 1977 (New York US) J.P.Murgo et al: "Physiologic signal acquisition and processing for human hemodynamic research in a clinical cardiac-catheterization laboratory" pages 696-702

## Description

La présente invention concerne un dispositif de mesure de la pression d'un liquide injecte dans un milieu compliant, tel un compartiment liquidien type articulaire ou un disque intervertébral, humain ou animal.

Chez les patients souffrant de lombo-radiculalgie par hernie discale, se pose encore aujourd'hui le problème du choix du traitement à proposer.

L'examen clinique et les examens para-cliniques pratiques ne sont pas à eux seuls suffisants, dans la majorite des cas, pour orienter à coup sur vers une décision thérapeutique appropriée.

La discomanométrie associée a l'étude de la tolérance discale au remplissage est une nouvelle méthode d'investigation complémentaire pré-opératoire basée sur la métrologie. Elle consiste, dans le but de déterminer l'état de détérioration discale et par là guider vers le choix thérapeutique le plus adéquat, à étudier le comportement physique du disque pathologique en analysant l'évolution des pressions intradiscales induites, pendant un temps déterminé, après injection soit de sérum salé isotonique, soit d'un produit de contraste radiologique, soit d'un mélange des deux.

La mise au point de cette nouvelle méthode à pu être réalisée grâce a l'étude d'un appareillage spécialisé appelé discomanomètrie( DMM ).

Les appareillages traditionnels de mesure de pressions physiologiques ont été essentiellement développés autour de la mesure des pressions hémodynamiques (normes NF c 74010 - 74300 74380 - 74382 ). Leurs caractéristiques et spécifications ne sont pas adaptées a l'utilisation envisagée.

Le dispositif, discomanomètre ( DMM ), selon l'invention permet de remédier à ces inconvénients.

Il est constitué de trois sous-ensembles ou parties distinctes:
1 ° - Un capteur/transducteur transformant le signal mécanique de pression appliqué sur sa membrane par le fluide, en un signal électrique utilisable par la deuxième partie du dispositif. 2_{°}- Un amplificateur/conditionneur du signal délivré par la première partie.
3_{°}- Un système d'enregistrement permettant d'obtenir le tracé de l'évolution du signal dans le temps.

Ces trois sous-ensembles S1, S2, et S3 sont définis dans la revendication 1.

Le sous-ensemble (S1) est de préférence constitué d'un capteur a jauge de contrainte ou d'un dispositif équivalent à usage médical (asepsie) pouvant mesurer, au minimum, des pressions variant de 0 à 10⁶ Pa (7.600 mm Hg) ou plus, pouvant supporter des surpressions allant jusqu'à 1,5. 10⁶ Pa (11.400 mm Hg) sans altération de ses caractéristiques d'origine, avec une linéarité < 0,5% PE et une erreur de répétabilité < 0,1 % PE.

Le sous-ensemble (S2) est de préférence constitué des éléments suivants:
- une alimentation isolée pour le capteur et le pré-amplificateur.
- un pré-amplificateur isolé, dit flottant, recevant les signaux du capteur. Les spécifications selon les normes en vigueur de ces dispositifs permettent d'assurer la sécurité élèctrique du patient exploré.
- une chaîne analogique de conditionnement du signal délivré par le pré-amplificateur comportant un ou plusieurs étages de normalisation du dit signal en fonction de la sensibilité du capteur et de l'admissibilité de l'enregistreur. Le taux d'amplification peut être variable continûment ou par bonds ou les deux simultanément. Un signal étalon peut être incorporé afin de calibrer les signaux enregistrés. Ce signal peut être complexe et être en relation directe avec les gammes de mesures si l'appareil en comporte plusieurs. La chaîne comporte un circuit de compensation de la balance du capteur, soit potentiométrique, soit électronique.

Un circuit d'affichage des valeurs de pression comportant une mémorisation, initialisée par l'opérateur grâce à une commande manuelle, permet d'indiquer la pression à To -action sur la commande- et, automatiquement affiche la valeur à T₆a. (To et T₆₀ exprimes en secondes).

Selon une variante la chaîne analogique de conditionnement peut être remplacée par une chaîne numérique de traitement du signal comportant un ou plusieurs des éléments suivants: microprocesseur et ses circuits associés, mémoires du programme de traitement, mémoires de travail, contrôleurs de communications, horloge, clavier alphanumérique, circuits d'interfaçage, convertisseur analogique/digital, convertisseur digital/analogique, écran ou dispositif d'affichage, disquette ou disque dur de stockage.

Le sous-ensemble (S3) est de préférence constitue par un enregistreur galvanométrique à stylet chauffant et papier thermographique, d'une plage d'inscription de 40 à 100 mm de large, la vitesse de défilement pouvant être réglée entre 2,5 et 10 mm/s.

Une variante de ce sous-ensemble peut être constituée d'un enregistreur à peigne thermique ("thermal array recorder"), d'une plage d'inscription de 40 à 200 mm de large, associant les enregistrements de signaux analogiques, alphanumériques et graphiques.

Selon une forme de réalisation préférentielle, les sous-ensembles (S2) et (S3)sont intégrés dans un même boîtier comportant une alimentation basse tension, afin de constituer un appareil homogène et pratique. Le boîtier peut posséder ou ne pas posséder de poignée pour le transport.

Les dessins et schémas annexés illustrant un mode de réalisation d'un dispositif conforme à la présente invention, sont donnés à titre d'exemples non limitatifs.
La figure 1 représente le dispositif dans son contexte d'utilisation.
La figure 2 représente un synoptique général des trois sous-ensembles (S1), (S2), (S3) composant le dispositif.
La figure 3 représente un modèle de capteur/transducteur utilisable comme sous-ensemble (S1), ainsi que son schéma fonctionnel élèctrique.
La figure 4 représente le schéma fonctionnel du sous-ensemble (S2).
La figure 5 représente le schéma fonctionnel d'une variante du sous-ensemble (S2).
La figure 6 représente le schéma fonctionnel du sous-ensemble (S3).
La figure 7 représente le schéma fonctionnel d'une variante du sous-ensemble (S3).
La figure 8 représente les courbes de pressions obtenues dans un disque intervertébral : (a) sain et normal, (b) pathologique du 1"' groupe (peu dégénéré), (c) pathologique du 2""" groupe (dégénéré), (d) pathologique du 3""" groupe (très dégénéré ou disque "bulging"), (e) pathologique du 4"' groupe (disque rompu).

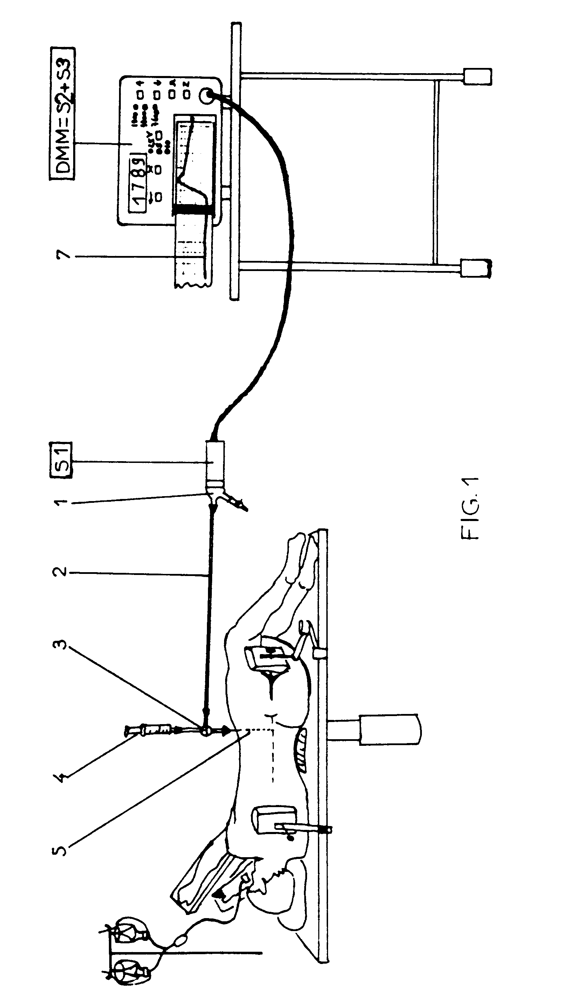

Le dispositif représenté sur la figure 1 comporte l'entrée du dôme du capteur (1) reliée à un cathéter prolongateur (2) branché sur une entrée d'un robinet à trois voies (3). Une seringue contenant le produit à injecter (4) est connectée sur la deuxième entrée du robinet. La sortie du robinet est raccordée à une aiguille, un trocart ou un cathéter (5) placé dans le disque intervertébral par ponction percutanée. Le capteur (S1) est raccordé au discomanomètre ( DMM ). Il permet d'obtenir le tracé du signal de pression (7).

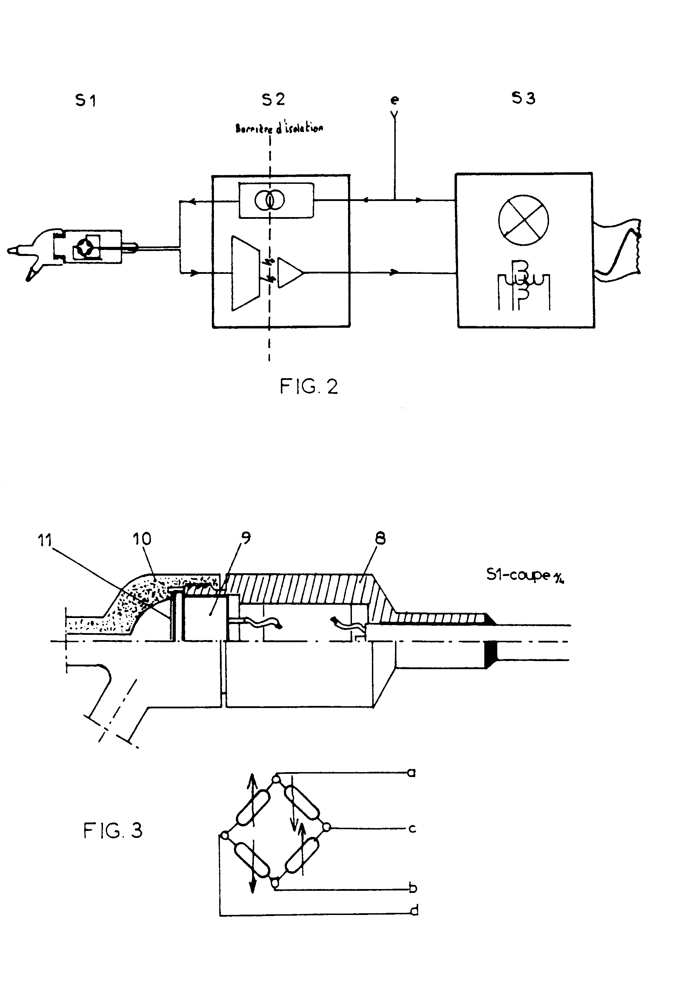

La figure 2 montre la disposition et l'interconnexion des sous-ensembles (S1), (S2) et (S3).

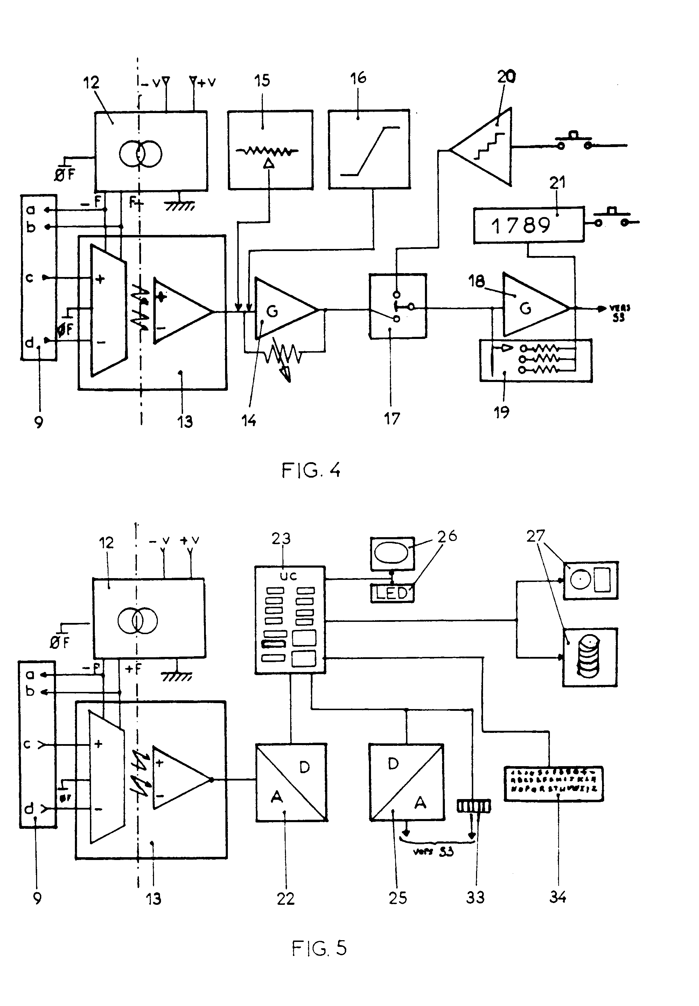

La figure 3 montre un capteur/transducteur (S1) comportant un corps en matière isolante (8) sur une extrémité duquel une cellule à jauge de contrainte (9) est disposée selon un montage étanche. Cette même extrémité est filetée sur une portion de sa longueur afin d'y adapter un dôme stérile à usage unique (10) en matière moulée. Ce dôme comporte une fine membrane en polyéthylène (11) qui est appliquée sur la cellule.Elle a pour rôle d'isoler, d'une manière aseptique, le patient du capteur.

La jauge de contrainte disposée en pont de Wheatstone possède deux entrées d'alimentation (a,b), deux sorties du signal (c,d).

Selon une forme de réalisation, le sous-ensemble (S2), représenté par la figure 4, comporte une alimentation (12) isolée galvaniquement à très faibles courants de fuites. Ce module (12) fournit l'énergie nécessaire à la jauge de contrainte (9) et au pré-amplificateur isolé (13) afin d'assurer leur fonctionnement. La sortie du pré-amplificateur (13) est reliée à l'entrée d'un amplificateur intermédiaire (14) à gain réglable,cet étage recevant la consigne de balance de zéro du capteur (tension d'équilibrage), produite soit par le potentiomètre (15) soit par le circuit (16) de compensation électronique. Le signal calibré et compensé est appliqué, via un circuit d'aiguillage (17), à l'étage final. Cet étage comporte un amplificateur (18) à gain réglable par bonds (19) permettant d'obtenir plusieurs gammes de mesures; trois dans cette forme.Le circuit (17) aiguille les signaux en provenance du circuit(14) et du circuit d'étalonnage(20); celui-ci permet la cali- bration du trace obtenu sur l'enregistreur. Ce circuit est actionné à la demande par une commande située sur la face avant du dispositif.

Un circuit d'affichage(21) reçoit le signal du (18). Selon un procédé du dispositif, une commande manuelle actionnée par l'opérateur, à un moment qu'il définit en observant le tracé en cours d'enregistrement, bloque l'afficheur sur la valeur de pression à l'instant To jusqu'à T₆₀ (exprimés en secondes). La valeur de la pression à cet instant est de nouveau affichée.

Le signal obtenu en sortie du circuit (18) est envoyé au sous-ensemble (S3).

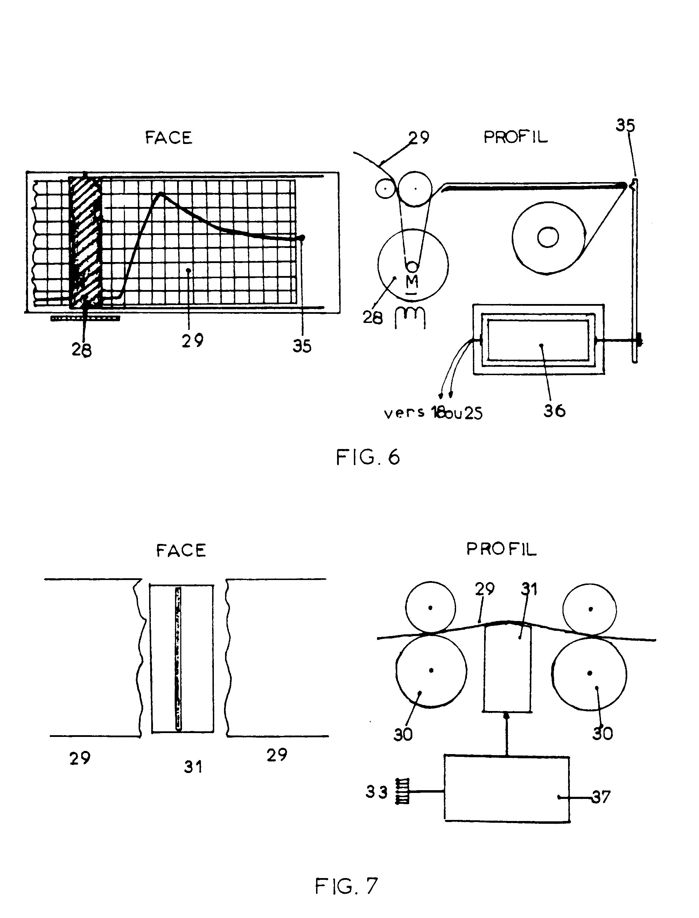

Selon une variante, représentée par la figure 5, le sous-ensemble (S2) comporte une alimentation (12) et un pré-amplificateur (13) comme décrits précédemment.

Le signal en provenance du (13) est appliqué à un convertisseur analogique/digital (22). Un organe de calculs architecturé autour d'un micro-ordinateur ou d'un microprocesseur (23), traite les données en provenance du (22) et les restitue, soit sous forme analogique à travers un convertisseur digital/analogique (25), soit sous forme numérique (33), selon l'option retenue pour le sous-ensemble (S3). Un clavier alphanumérique (34) permet de commander le dispositif et de dialoguer avec le (23). De cette manière l'identification du patient, les données cliniques et les calculs effectués peuvent être enregistrés sur le même document que les courbes de pression. Un écran de contrôle et de dialogue (26), optionnel, qui peut être constitué soit par un moniteur vidéo, soit par un afficheur à cristaux liquides, permet de faciliter l'interaction avec le dispositif. Une mémoire de masse (27), optionnelle, qui peut être constituée d'une disquette ou d'un disque dur permet le stockage des informations pour un traitement statistique ultérieur.

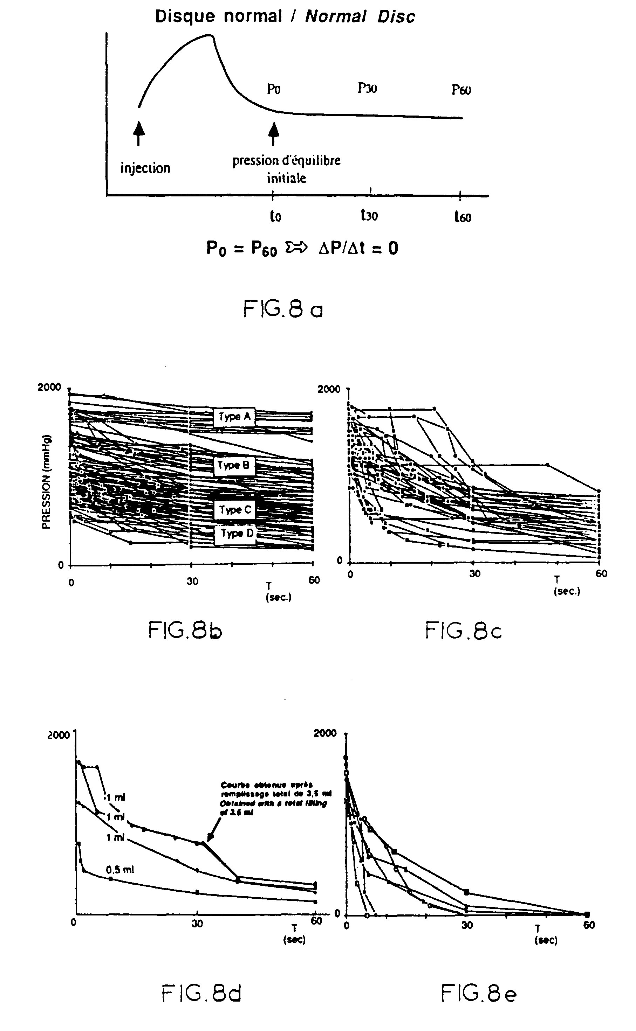

Le sous-ensemble (S3), représenté par la figure 6, comporte une cinématique (28) assurant le défilement du papier thermographique (29) sous un stylet chauffant (35) mobile axialement reproduisant ainsi le tracé du signal de pression. Le stylet (35) est solidaire d'un galvanomètre à cadre mobile (36) traduisant le signal élèctrique qu'il reçoit du (18) ou du (25) en énergie mécanique. Les vitesses sélec- tables par l'opérateur sont au nombre de trois : 2,5 - 5 et 10 mm/s.

Selon une variante, le sous-ensemble (S3), représenté par la figure 7, est constitué d'une cinématique (30) assurant le défilement du papier thermographique (29), aux mêmes vitesses que ci- dessus, devant la matrice d'un peigne thermique (31) disposé en ligne, sur toute la largeur du papier, au pas de 8 points par millimètre, chaque point pouvant être commandé sélectivement. Une électronique de contrôle (37) reçoit les informations en provenance de (33) et pilote le peigne. Ce procédé, tout en éliminant les défauts de linéarité et de bande passante du système galvanométrique, possède l'avantage d'être directement compatible avec les systèmes numériques et, de plus, autorise l'inscription de caractères alphanumériques superposés aux tracés graphiques.

La réunion de ces trois sous-ensembles ou de leurs variantes constitue le dispositif selon l'invention qui est particulièrement destiné à usage médical, pour l'application d'une méthode métrologique, notamment à la mesure des pressions d'un liquide injecté dans un milieu compliant, tel un compartiment liquidien type articulaire ou un disque intervertébral, humain ou animal, pour en étudier le comportement physique à des fins diagnostiques dans un but thérapeutique.

## Revendications

1. Dispositif de mesure de la pression d'un liquide, injecté dans un compartiment liquidien type articulaire ou un disque intervertébral humain ou animal, en vue notamment de l'aide au diagnostic médical et au meilleur choix thérapeutique des lombalgies ou lombo-radiculalgies par hernie discale, constitué par la réunion de trois sous-ensembles S1, S2, S3, formant un tout appelé discomanomètre (DMM). Chaque sous-ensemble étant constitué respectivement :
- pour S1; par un capteur/transducteur à jauge de contrainte, relié à une aiguille, un trocart ou un cathéter susceptible d'être placé dans le compartiment ou le disque par ponction percutanée, dont la sensibilité permet de mesurer des pressions induites variant de 0 à 10⁶ Pa (7.600 mm Hg).
- pour S2; par un manomètre électronique qui amplifie et conditionne le signal déli- vréparS1.
- pour S3; par un enregistreur graphique traduisant les signaux électriques élaborés par S2, en documents exploitables dans un but d'aide au diagnostic médical.

2. Dispositif selon la revendication 1, caractérisé en ce que le manomètre électronique amplificateur/conditionneur S2, relié en amont à S1 et en aval à S3, comporte une alimentation (12) et un pré-amplificateur (13) flottants du point de vue électrique par rapport au reste du système (isolation galvanique). Leurs très faibles courants de fuites assurant la sécurité du patient.

3. Dispositif selon la revendication 2, caractérisé en ce que le signal issu du pré-amplificateur (13) est conditionné soit par un amplificateur analogique, soit par un système numérique.

4. Dispositif selon la revendication 3, caractérisé par un amplificateur analogique comportant un étage intermédiaire à gain réglable (14), recevant la tension d'équilibrage produite par un potentiomètre (15) ou par un circuit de compensation électronique (16). Un circuit d'aiguillage (17) reçoit les signaux en provenance du circuit (14) et du générateur d'étalonnage (20), pour les appliquer à l'étage final constitué par un amplificateur (18) à gain réglable par bonds (19) permettant d'obtenir plusieurs gammes de mesures. Le signal délivré par (18) est appliqué à S3.

5. Dispositif selon la revendication 4, caractérisé par un circuit d'affichage des valeurs de la pression, qui à un moment défini par l'opérateur, mémorisera le valeur obtenue à cet instant To jusqu'à T₆₀ (en secondes), pour afficher la nouvelle valeur du moment.

6. Dispositif selon la revendication 3, caractérisé par un système numérique comportant un convertisseur analogique/digital (22) qui reçoit le signal délivré par le circuit pré-amplificateur (13). Un organe de calcul, architecturé autour d'un micro-ordinateur ou d'un micro-processeur (23), traite les données émises par le circuit (22) et les restitue soit sous forme analogique à travers un convertisseur digital/analogique (25), soit sous forme numérique (33). Les signaux issus de (25) ou (33) sont appliqués à S3.

7. Dispositif selon la revendication 6, caractérisé en ce qu'il comporte un clavier numérique ou alpha-numérique (34), permettant l'introduction des commandes du discomanomètre (DMM), et des données cliniques concernant le patient devant figurer sur l'enregistrement.

8. Dispositif selon la revendication 6, caractérisé par une disquette ou un disque dur magnétique, remplissant le rôle de mémoire de masse permanente, permettant le stockage des informations pour un traitement statistique ultérieur.

9. Dispositif selon l'une des revendications 1, 2, 4 ou 6, caractérisé en ce qu'il est constitué soit par un enregistreur galvanométrique, soit par un enregistreur à peigne thermique ("thermal array recorder"), l'enregistreur disposant d'une ou plusieurs vitesses de défilement.

10. Dispositif selon la revendication 1, caractérisé en ce que S2 et S3 sont intégrés dans un même boîtier.

11. Dispositif selon la revendication 4, caractérisé en ce que les gammes de mesures sont 2,5 x ₁₀^{5 -} ₅ x 10⁵ et 10⁶ Pa (1.900 3.800 et 7.600 mm Hg).

12. Dispositif selon la revendication 7, caractérisé en ce qu'un écran de contrôle et de dialogue (26), constitué d'un moniteur vidéo ou d'un écran à cristaux liquides, permet de faciliter l'interaction avec le dispositif.

13. Dispositif selon la revendication 9, caractérisé en ce que les vitesses de défilement sont 2,5 - 5 et 10 mm/s.

## Claims

1. Pressure measurement device of a liquid, injected into an articular compartment or an intervertebral disc human or animal,with in particular a view to medical diagnosis aiding to and to the best therapeutic choice for lumbago or sciatica by discal hernia, it is made of the union of three sub-units S1, S2 and S3 named DISCOMANOMETER (DMM). The sub-units can be described as follows :
- S1 is a constraint gauge transducer linked to a needle, a trocart or catheter likely to be put in the compartment or disc by percutaneous puncture, whose sensitivity allows to measure induced pressures from Zero to 1 x 10⁶ Pa (7,600 mm Hg).
- S2 is an electronic manometer which amplify and condition the signal sent by S1.
- S3 is a graphic recorder translating the electric signals given by S2 into readable documents to be used as an help to medical diagnosis.

2. Device according to claim #1, characterized in what the electronic amplifier /conditioner manometer S2 input connected to S1, and output connected to S3 includes a power supply (12) and a preamplifier (13) both of them electrically floating in comparison with the remains of the system (galvanic insulation). Their very low leakage currents assuring the patient safety.

3. Device according to claim #2, characterized in what the signal coming from the preamplifier (13) is conditioned either by an analog amplifier or a digital system.

4. Device according to claim #3, characterized by an analog amplifier including an intermediate stage (14) with an adjustable amplification rate, receiving the balanced voltage given by either a potentiometer (15) or a offset compensation electronic circuit. A routing circuit (17) receives signals coming from circuit (14) and from reference circuit (20), in order to apply them to the final stage made of an step by step (19) adjustable amplifier (18) allowing to get several measurement ranges. The output signal from (18) is sent to S3.

5. Device according to claim #4, characterized by a display device showing the pressure values, which at a time chosen by the operator, will be continuously displaying the obtained pressure value at that time To, until T₆ₒ (in seconds) where the new pressure value will be displayed.

6. Device according to claim #3, characterized by a digital system including an A/D converter (22) receives the signal sent by preamplifier circuit (13). Data coming from (22) are treated thru a computing device (23) and resent either as an analog signal through a D/A converter (25) or as a digital signal (33). Signals coming from (25) or (33) are applied to S3.

7. Device according to claim #6, characterized in what it includes a keyboard (34) either numeric or alphanumeric allowing to inputs the Discomanometer (DMM) commands and clinical data about the patient to be added to the graph.

8. Device according to claim #6, characterized by a diskette or hard-disk drive is used as a mass memory allowing data storage for future statistical treatment.

9. Device according to any claims #1; #2; #4 or #6 characterized in what it includes either a galvanometric recorder or a thermal array recorder having one or several paper speeds.

10. Device according to any claims #1, characterized in what S2 and S3 belong to the same box.

11. Device according to any claims #4, characterized in what the measure ranges are 2.5 x 10⁵; 5 x 10⁵ et 1 x 10⁶ Pa (1,900 ; 3,800 and 7,600 mm Hg)

12. Device according to any claims #7, characterized in what a control and dialog screen (26), made of either a video monitor or LCD, allows to make easier the interaction with device.

13. Device according to any claims #9, characterized in what the paper speeds are 2.5; 5 et 10 mm/s.

## Patentansprüche

1. Die Vorrichtung für das Messen des Drucks einer Flüssigkeit, die in einen flüssigen Bereich der Gelenke oder eine intervertebrale menschliche oder tierische Scheibe eingespritzt wird, insbesondere im Hinblick auf ärztliche Diagnose und bessere therapeutische Wahl bei Lomalgien oder Lombo-Radiculalgies bei Bandscheibenvorfall, setzt sich aus drei Teilmontagen S1, S2 und S3 zusammen, als Ganzes "Discomanometer" bezeichnet (DMM). Jede Teilmontage wird jeweils wie folgt dargestellt:
- für S1 : durch eine Messzelle des Spannungsmessgerätes, die mit einer Nadel, einem Trokar oder einer Leitungssonde verbunden ist und geignet ist, im Bereich oder der Scheibe durch Hautpunktion angebracht zu werden, deren Sensibilität es zulässt, den induzierten Druck zu messen, der von 0 bis 10⁶ Pa (7.600 mm Hg) variiert.
- für S2 : durch einen elektronischen Manometer, der das durch S1 gelieferte Signal verstärkt und bedingt.
- für S3 : durch ein grafisches Registriergerät, das die durch S2 verarbeiteten elektrischen Signale in nutzbare Dokumente mit dem Ziel der ärztlichen Diagnose übersetzt.

2. Vorrichtung gemäß der Anforderung 1, karakterisiert durch den elektronischen Manometer-Verstärker S2, oberhalb mit S1 und unterhalb mit S3 verbunden, enthält eine Zufuhr (12) und einen beweglichen Verstärker (13) aus Sicht der Elektrik im Vergleich zum Rest des Systems ( galvanische Isolierung). Ihre sehr schwache Entweichungsströmung gewährleistet die Sicherheit des Patienten.

3. Vorrichtung gemäß der Anforderung 2, karakterisiert durch das Signal des Vorverstärkers (13), wird sowohl durch den analogischen Verstärker bedingt als auch durch ein numerisches System.

4. Vorrichtung gemäß der Anforderung 3, karakterisiert durch den analogischen Verstärker, umfasst eine Zwischenstufe zur Gewinnregulierung (14), empfängt die Spannung des erzeugten Gleichgewichts durch einen Potentiometer (15) oder durch den elektronischen Ausgleichskreislauf (16). Ein Steuerungskreislauf (17) empfängt die Signale aus Richtung des Kreislaufs (14) und des Eichgenerators (20), um sie auf die Endstufe zu lenken, die sich aus dem Verstärker (18 ) zur Gewinnregulierung durch Sprünge ( 19 ) zusammensetzt und es erlaubt, mehrere Messbereiche zu erreichen. Das durch (18) ausgelöste Signal wird auf S3 gelenkt.

5. Vorrichtung gemäß der Anforderung 4, karakterisiert durch einen Anzeigekreislauf des Druckwertes, der zu einem vom Operator bestimmten Zeitpunkt den erreichtent Wert speichern wird, To bis T₆₀ (in Sekunden), um den neuen momentanen Wert bekanntzugeben.

6. Vorrichtung gemäß Anforderung 3, karakterisiert durch ein numerisches System, umfasst einen Analog-Digital-Umsetzer (22), der das durch den Vorverstärkerkreislauf (33) ausgelöste Signal empfängt. Ein Digitalbrechner des Mikrokomputers oder des Mikroprozessors (23) verarbeitet die Daten aus dem Kreislauf (22) und gibt sie wieder sowohl in analogischer Form (25) über einen Digital-Analog-Umsetzer als auch in numerischer Form. (33). Die Signale, die aus (25) und (33) hervorgehen, werden an S3 gerichtet.

7. Vorrichtung gemäß Anforderung 6, karakterisiert durch eine numerische oder alphanumerische Tastatur (34) ; gestattet die Einführung des Discomanometers (DMM) und der klinischen Daten bezüglich des Patienten welche in der Speicherung erscheinen müssen.

8. Vorrichtung gemäß Anforderung 6, karakterisiert durch eine Diskette oder eine magnetische Speicherplatte, erfüllt die Rolle des ständigen Massenspeichers und ermöglicht eine Speicherung der Daten für eine spätere Verarbeitung.

9. Vorrichtung gemäß einer der Anforderungen 1,2,4 oder 6, dadurch karakterisiert, daß sie sich sowohl aus einem galvanometrischen Registriergerät als auch aus dem thermischen Messrechen zusammensetzt, da das Registriergerät über eine oder mehrere Abspulgeschwindigkeiten verfügt.

10. Vorrichtung gemäß der Anforderung 1, dadurch karakterisiert, daß S1 und S3 in demselben Gehäuse sind.

11. Vorrichtung gemäß der Anforderung 4, karaterisiert durch die Messbereiche 2,5 x 10^{5 -} 5 x 10⁵ und 10⁶ Pa (1900, 3800 und 7600 mm Hg)

12. Vorrichtung gemäß der Anforderung 7, karakterisiert durch einen Kontroll- und Kommunikationsbildschirm, der aus einem Monitor oder Bildschirm aus flüssigem Kristall besteht und es erlaubt, die Wechselwirkung mit der Vorrichtung zu erleichtern.

13. Vorrichtung gemäß der Anforderung 9, karakterisiert durch die Abspulgeschwindigkeiten von 2,5 - 5 und 10 mm/s.
